# EUROPEAN PATENT APPLICATION

(11) **EP 4 338 749 A1**
(43) Date of publication of application: **20.03.2024**
(21) Application number: 22195738.4
(22) Date of filing: 14.09.2022
(51) Int. Cl.: A61K 39/02, A61P 1/04

(54) **VACCINE AGAINST HELICOBACTER PYLORI INFECTION**

(71) Applicant: Iguana Biotechnology GmbH, 80331 Munich (DE)
(72) Inventor: KALALI, Behnam, 80939 Munich (DE); WEDERSHOVEN, Volker, 81667 Munich (DE)
(74) Representative: Isarpatent

(57) **Abstract**

Present application relates to a vaccine comprising a polypeptide with multiple epitopes from UreB, FliD, HpaA, Hp0231, NapA, BabA for use in preventing or treating a disease caused by *Helicobacter pylori* (*H. pylori*) adjuvanted with flagellin or CTB and MVA as vector.

## Description

### Technical field

The present invention relates to a polypeptide, a polypeptide for use in preventing or treating an infection by Helicobacter pylori (*H. pylori)* and/or a disease caused by *H. pylori* infection, and also relates to a composition comprising said polypeptide, and a composition for use in preventing or treating an infection by Helicobacter pylori (*H. pylori)* and/or a disease caused by *H. pylori* infection.

### Background

The *H. pylori* infection is the most common bacterial infectious disease in humans: half of the global population is infected with this bacterium. The colonisation of *H. pylori* in the human stomach causes gastritis (in all patients infected, see Blaser MJ. Hypothesis: the changing relationships of Helicobacter pylori and humans: implications for health and disease. J Infect Dis. 1999;179:1523-1530), gastric and duodenal ulcers (in approximately 20% of patients) and gastric carcinoma (in 1% of patients). These diseases are associated with a high morbidity and mortality rate. Each year 1.100,000 people develop gastric carcinoma worldwide, including over 150,000 in Europe and approximately 20,000 in Germany. Gastric cancer is associated with considerable socio-economic costs. Treating a single patient with gastric cancer currently costs about EUR 50.- 80.000.
- Prevention of gastric cancer includes early treatment of an infection caused by *H. pylori.* According to the guidelines, all of individuals with an infection caused by *H. pylori* requires treatment (Malfertheiner P et al. European Helicobacter and Microbiota Study group. Management of Helicobacter pylori infection: the Maastricht VI/Florence consensus report. Gut. 2022 Aug 8:gutjnl-2022-327745. doi: 10.1136/gutjnl-2022-327745. Epub ahead of print. PMID: 35944925. DOI: 10.1136/gutjnl-2022-327745). At present, it is difficult to predict which patients will develop the subsequent diseases associated with an *H. pylori* infection. Based on the results of numerous studies, general treatment of the *H. pylori* infection to prevent related disorders like gastric and duodenal ulcers or gastric carcinoma is cost efficient as it would prevent over 95% of cases (Graham DY, Shiotani A. The time to eradicate gastric cancer is now. Gut 2005;54:735-738). Therapy is clearly indicated for patients with gastric ulcers, precancerous or definitive gastric cancer, relatives of gastric cancer patients as well as patients requiring long-term therapy with non-steroidal anti-inflammatory drugs (including aspirin for cardiovascular diseases). Although antibiotic resistance is steadily increasing, the treatment of all individuals infected with *H. pylori* is recommended in Japan (Shiota S, Murakami K, Fujioka T, Yamaoka Y. Population-based strategies for Helicobacter pylori-associated disease management: a Japanese perspective. Expert Rev Gastroenterol Hepatol. 2010 Apr;4(2):149-56).

The standard first line therapy of infections caused by *H. pylori* to date consists of two antibiotics combined with a proton pump inhibitor such as omeprazole. The cost of a oneweek treatment is approximately EUR 200,00 per patient. This therapy has significant sideeffects in some patients and leads to a steep increase in resistant pathogens. Because secondand third-line therapies often fail, about 10% of all patients cannot be further treated.

Therefore, if a vaccine against *H. pylori* were available, it would benefit millions of patients and reduce healthcare costs. A global survey among gastroenterologists represented in 5 working groups demonstrated 100% acceptance of a *H. pylori* vaccine (Maastricht VI WG 4, statement 27; Malfertheiner P, Megraud F, Rokkas T, et al. Gut 2022;71:1724-1762).

### Brief description of the invention

The goal of the project is to provide a polypeptide and a composition comprising the same which can be used as a therapeutic and/or a prophylactic vaccine against *H. pylori,* the most common cause of a chronic bacterial infection and the main cause for gastric ulcers and gastric cancer - the third most common type of cancer with a mortality of 800.000 patients worldwide in 2011 (Global cancer statistics. Jemal A, et al. CA Cancer J Clin. 2011;61(2):69)-, duodenal ulcer disease and Mucosa Associated Lymphoid Tissue (MALT) lymphomas.

Vaccines are usually manufactured from inactivated pathogens or pathogen fragments (e.g. envelope proteins) that enable the immune system to identify and eliminate pathogens. However, this mechanism is only successful when the proper immune response consisting of the humoral and cellular responses reach the anatomical location of infection. Due to the difficult accessibility of the mucosa for the immune compartments circulating in the blood, it is fundamentally of importance that the vaccine against *H. pylori* stimulates the immune system with mucosal presence.

The ideal points for attack are bacterial proteins that are essential for survival, infectiousness or disease pathogenesis. The vaccine disclosed in the present invention provides immunisation against *H. pylori* using a complex of epitopes from different antigens.

In this regard, the inventors found that flagellin, the ligand of TLR5, can induce mucosal immune responses in the gastrointestinal tract (doi.org/10.1016/j.mad.2008.01.009; DOI: 10.1016/j.vaccine.2003.12.035). In addition, there are well demonstrated observations reporting the efficacy of a double mutant variant of the heat labile toxin of *E*. *coli* (dmLT) in induction of immune response with mucosal localization (Clements JD, Norton EB. The Mucosal Vaccine Adjuvant LT(R192G/L211A) or dmLT. mSphere. 2018 Jul 25;3(4):e00215-18).

In addition, recent studies confirmed a striking success of T- and B- cell response in the GI tract using modified viruses (e.g. Modified Vaccinia Virs Ankara; MVA) and viral like particles (DOl: 10.1186/s12985-019-1212-y; doi.org/10.1186/s12865-022-00494-4). MVA as vector vaccine offers several advantages for the development of recombinant vaccine candidates. In addition to various host-restriction genes, MVA lacks several immunomodulatory genes of which some have proven to be quite efficient in skewing the immune response in an unfavourable way to control infection in the host. Special viral like particles possess well exposed loops that have the capacity for carrying even big size protein molecules. They can not only tolerate the extreme acidic environment of the stomach, but also induce the immune system in the GI mucosa.

Reference strains isolated from patients showing activity in these tests were used to obtain inhibitory components for purification by multi-stage fractionation such that they can be identified using mass spectrometry. Prospective targets were then prioritized based on data base, literature and patent searches. The most promising candidates were qualified for further preclinical development by immunisation studies in animals.

Present invention provides a vaccine against *H. pylori* which consists of a complex of epitopes from various key antigens, potentially inducing T-cell and B-cell response and a mucosal adjuvant. Earlier immunisation approaches against *H. pylori* have targeted structures within or on the surface of the bacterium that are not essential for the pathogen (Aebischer T, et al. Correlation of T cell response and bacterial clearance in human volunteers challenged with H. pylori revealed by randomized controlled vaccination with Ty21a-based Salmonella vaccines. Gut 2008; 57: 1065-1072). Immunisation that induces an immune response against an essential protein seems far more promising. On the other hand, a vaccine against a complex microorganism like H. pylori using one or two essential antigen was until now not successful. Therefore, this invention targets more than only 2 antigens. Apart from challenging issues of production of big antigenic molecules, to overcome the problematic of undesired side effect using large antigens, only the selected epitopes of the target antigens are identified and fused together via certain linkers. This approach has not been applied to date as part of the relevant proteins were found only recently.

The target proteins used by the present inventors are listed below:
- Urease B (UreB): A secreted protein also found on the surface of the bacterium. UreB is a part of the enzyme Urease which is a virulence factor and conserved among *H. pylori* family.
- HpaA: It is a surface conserved virulence factor of *H. pylori.* Said protein is a potent T-cell antigen and is highly conserved and essential for *H. pylori* to colonise. As a cell surface protein, it is easily accessible to the immune system, i.e., immunogenic, and induces a robust T-cell response in immunisation. The interplay between the neutralisation of the YGT and the simultaneous induction of an HpaA-specific cellular response leads to successful vaccination against H. pylori
- FliD: is a flagellar conserved antigen and belongs to the virulence factors of *H. pylori.*
- Hp0231: This antigen is presented on the surface of the bacterium and belongs to the virulence factors.
- NapA: is a known secreted conserved virulence antigen.
- BabA: It is a surface protein, conserved among H. pylori's family and belongs to the virulence factors.

According to comprehensive bioinformatic analysis of the above-mentioned proteins, a list of epitopes was identified. Hereby, B-cell specific epitopes as well as T-cell epitopes -presented via MHC-I and MHC-II molecules- were included.

The presently disclosed polypeptide MEU is used to mix with an adjuvant or said MEU is fused with an adjuvant to attain desired effect. The presently disclosed vaccine is intensively optimised in mouse models, e.g. oral priming and/or systemic boost, optimal B- and T-cell activation, and therefore will promise greater efficacy than conventional formulations.

### Detailed description of the invention

Throughout this description and the following claims, unless the context requires otherwise, the term "comprise", "comprises" or "comprising", should be understood as the inclusion of a stated member, integer, or step but not the exclusion of any other non-stated member, integer or step. The term "consisting of" is a particular embodiment of the term "comprise", indicating that any other non-stated member, integer, or step is excluded. In the present invention, the term "comprise" encompasses the term "consist of". The term "comprising" encompasses "including" as well as "consisting", for example, a composition "comprising" X may consist exclusively of X or may include something additional, for example, X + Y.

The terms "a" and "an" and "the" in the description should be understood as covering both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the description as if it were individually recited herein. No language in the description should be construed as indicating any non-claimed element essential to the practice of the present invention.

The term "and/or" in the present description should be taken as specific disclosure of each of the two specified features or components with or without the other. For example, "A and/or B" is to be taken as specific disclosure of each of (i) A, (ii) B and (iii) A and B, just as if each is set out individually herein.

The first aspect of the present invention relates to a polypeptide comprising a sequence of an epitope from UreB, wherein said sequence of an epitope from UreB comprises a sequence exhibiting at least 70%, 80%, 90% or exhibiting 100% identity to SEQ ID NO: 1, and/or a sequence exhibiting at least 70%, 80%, 90% or exhibiting 100% identity to SEQ ID NO: 2, and/or a sequence exhibiting at least 70%, 80%, 90% or exhibiting 100% identity to SEQ ID NO: 3, and/or a sequence exhibiting at least 70%, 80%, 90% or exhibiting 100% identity to SEQ ID NO: 4.

The following two or more preferred embodiments can be combined according to the effects desired to be achieved.

Preferably, above polypeptide in the present invention comprises a sequence of an epitope from UreB, wherein said sequence of an epitope from UreB comprises a sequence exhibiting at least 70%, 80%, 90% or exhibiting 100% identity to SEQ ID NO: 1, a sequence exhibiting at least 70%, 80%, 90% or exhibiting 100% identity to SEQ ID NO: 2, a sequence exhibiting at least 70%, 80%, 90% or exhibiting 100% identity to SEQ ID NO: 3, and a sequence exhibiting at least 70%, 80%, 90% or exhibiting 100% identity to SEQ ID NO: 4, more preferably, each of sequence exhibits 70%, 80%, 90%, or 100% to the corresponding SEQ ID NO.

Preferably, above polypeptide in the present invention comprises a sequence of an epitope from UreB, wherein said sequence of an epitope from UreB comprises a sequence of SEQ ID NO: 1, a sequence of SEQ ID NO: 2, a sequence of SEQ ID NO: 3, and a sequence of SEQ ID NO: 4.

Preferably, one of above polypeptides further comprises a sequence of an epitope from FliD, and wherein said sequence of an epitope from FliD comprises a sequence exhibiting at least 70%, 80%, 90% or exhibiting 100% identity to SEQ ID NO: 5, and/or a sequence exhibiting at least 70%, 80%, 90% or exhibiting 100% identity to SEQ ID NO: 6, and/or a sequence exhibiting at least 70%, 80%, 90% or exhibiting 100% identity to SEQ ID NO: 7, and/or a sequence exhibiting at least 70%, 80%, 90% or exhibiting 100% identity to SEQ ID NO: 8, and/or a sequence exhibiting at least 70%, 80%, 90% or exhibiting 100% identity to SEQ ID NO: 9, and/or a sequence exhibiting at least 70%, 80%, 90% or exhibiting 100% identity to SEQ ID NO: 10, and/or a sequence exhibiting at least 70%, 80%, 90% or exhibiting 100% identity to SEQ ID NO: 11, and/or a sequence exhibiting at least 70%, 80%, 90% or exhibiting 100% identity to SEQ ID NO: 12, and/or a sequence exhibiting at least 70%, 80%, 90% or exhibiting 100% identity to SEQ ID NO: 13, and/or a sequence exhibiting at least 70%, 80%, 90% or exhibiting 100% identity to SEQ ID NO: 14, and/or a sequence exhibiting at least 70%, 80%, 90% or exhibiting 100% identity to SEQ ID NO: 15. More preferably, one of above polypeptides further comprises a sequence of an epitope from FliD, and wherein said sequence of an epitope from FliD comprises a sequence exhibiting at least 70%, 80%, 90% or exhibiting 100% identity to SEQ ID NO: 5, a sequence exhibiting at least 70%, 80%, 90% or exhibiting 100% identity to SEQ ID NO: 6, a sequence exhibiting at least 70%, 80%, 90% or exhibiting 100% identity to SEQ ID NO: 7, a sequence exhibiting at least 70%, 80%, 90% or exhibiting 100% identity to SEQ ID NO: 8, a sequence exhibiting at least 70%, 80%, 90% or exhibiting 100% identity to SEQ ID NO: 9, a sequence exhibiting at least 70%, 80%, 90% or exhibiting 100% identity to SEQ ID NO: 10, a sequence exhibiting at least 70%, 80%, 90% or exhibiting 100% identity to SEQ ID NO: 11, a sequence exhibiting at least 70%, 80%, 90% or exhibiting 100% identity to SEQ ID NO: 12, a sequence exhibiting at least 70%, 80%, 90% or exhibiting 100% identity to SEQ ID NO: 13, a sequence exhibiting at least 70%, 80%, 90% or exhibiting 100% identity to SEQ ID NO: 14, and a sequence exhibiting at least 70%, 80%, 90% or exhibiting 100% identity to SEQ ID NO: 15, still more preferably, each of sequence exhibits 70%, 80%, 90%, or 100% to the corresponding SEQ ID NO. Further preferably, one of above polypeptides further comprises a sequence of an epitope from FliD, and wherein said sequence of an epitope from FliD comprises a sequence of SEQ ID NO: 5, a sequence of SEQ ID NO: 6, a sequence of SEQ ID NO: 7, a sequence of SEQ ID NO: 8, a sequence of SEQ ID NO: 9, a sequence of SEQ ID NO: 10, a sequence of SEQ ID NO: 11, a sequence of SEQ ID NO: 12, a sequence of SEQ ID NO: 13, a sequence of SEQ ID NO: 14, and a sequence of SEQ ID NO: 15.

Preferably, one of above polypeptides further comprises a sequences of an epitope from HpaA, and wherein said sequence of an epitope from HpaA comprises a sequence exhibiting at least 70%, 80%, 90% or exhibiting 100% identity to SEQ ID NO: 16, and/or a sequence exhibiting at least 70%, 80%, 90% or exhibiting 100% identity to SEQ ID NO: 17, and/or a sequence exhibiting at least 70%, 80%, 90% or exhibiting 100% identity to SEQ ID NO: 18, and/or a sequence exhibiting at least 70%, 80%, 90% or exhibiting 100% identity to SEQ ID NO: 19, and/or a sequence exhibiting at least 70%, 80%, 90% or exhibiting 100% identity to SEQ ID NO: 20, and/or a sequence exhibiting at least 70%, 80%, 90% or exhibiting 100% identity to SEQ ID NO: 21, and/or a sequence exhibiting at least 70%, 80%, 90% or exhibiting 100% identity to SEQ ID NO: 22. More preferably, one of above polypeptides further comprises a sequences of an epitope from HpaA, and wherein said sequence of an epitope from HpaA comprises a sequence exhibiting at least 70%, 80%, 90% or exhibiting 100% identity to SEQ ID NO: 16, a sequence exhibiting at least 70%, 80%, 90% or exhibiting 100% identity to SEQ ID NO: 17, a sequence exhibiting at least 70%, 80%, 90% or exhibiting 100% identity to SEQ ID NO: 18, a sequence exhibiting at least 70%, 80%, 90% or exhibiting 100% identity to SEQ ID NO: 19, a sequence exhibiting at least 70%, 80%, 90% or exhibiting 100% identity to SEQ ID NO: 20, a sequence exhibiting at least 70%, 80%, 90% or exhibiting 100% identity to SEQ ID NO: 21, and a sequence exhibiting at least 70%, 80%, 90% or exhibiting 100% identity to SEQ ID NO: 22, still more preferably, each of sequence exhibits 70%, 80%, 90%, or 100% to the corresponding SEQ ID NO. Further preferably, one of above polypeptides further comprises a sequences of an epitope from HpaA, and wherein said sequence of an epitope from HpaA comprises a sequence of SEQ ID NO: 16, a sequence of SEQ ID NO: 17, a sequence of SEQ ID NO: 18, a sequence of SEQ ID NO: 19, a sequence of SEQ ID NO: 20, a sequence of SEQ ID NO: 21, and a sequence of SEQ ID NO: 22.

Preferably, one of above polypeptides further comprises a sequences of an epitope from HP0231, and wherein said sequence of an epitope from HP0231 comprises a sequence exhibiting at least 70%, 80%, 90% or exhibiting 100% identity to SEQ ID NO: 23, and/or a sequence exhibiting at least 70%, 80%, 90% or exhibiting 100% identity to SEQ ID NO: 24, and/or a sequence exhibiting at least 70%, 80%, 90% or exhibiting 100% identity to SEQ ID NO: 25, and/or a sequence exhibiting at least 70%, 80%, 90% or exhibiting 100% identity to SEQ ID NO: 26, and/or a sequence exhibiting at least 70%, 80%, 90% or exhibiting 100% identity to SEQ ID NO: 27. More preferably, one of above polypeptides further comprises a sequences of an epitope from HP0231, and wherein said sequence of an epitope from HP0231 comprises a sequence exhibiting at least 70%, 80%, 90% or exhibiting 100% identity to SEQ ID NO: 23, a sequence exhibiting at least 70%, 80%, 90% or exhibiting 100% identity to SEQ ID NO: 24, a sequence exhibiting at least 70%, 80%, 90% or exhibiting 100% identity to SEQ ID NO: 25, a sequence exhibiting at least 70%, 80%, 90% or exhibiting 100% identity to SEQ ID NO: 26, and a sequence exhibiting at least 70%, 80%, 90% or exhibiting 100% identity to SEQ ID NO: 27, still more preferably, each of sequence exhibits 70%, 80%, 90%, or 100% to the corresponding SEQ ID NO. Further preferably, one of above polypeptides further comprises a sequences of an epitope from HP0231, and wherein said sequence of an epitope from HP0231 comprises a sequence of SEQ ID NO: 23, a sequence of SEQ ID NO: 24, a sequence of SEQ ID NO: 25, a sequence of SEQ ID NO: 26, and a sequence of SEQ ID NO: 27.

Preferably, one of above polypeptides further comprises a sequence of an epitope from NapA, and wherein said sequence of an epitope from NapA comprises a sequence exhibiting at least 70%, 80%, 90% or exhibiting 100% identity to SEQ ID NO: 28, and/or a sequence exhibiting at least 70%, 80%, 90% or exhibiting 100% identity to SEQ ID NO: 29. More preferably, one of above polypeptides further comprises a sequence of an epitope from NapA, and wherein said sequence of an epitope from NapA comprises a sequence exhibiting at least 70%, 80%, 90% or exhibiting 100% identity to SEQ ID NO: 28, and a sequence exhibiting at least 70%, 80%, 90% or exhibiting 100% identity to SEQ ID NO: 29, still more preferably, each of sequence exhibits 70%, 80%, 90%, or 100% to the corresponding SEQ ID NO. Further preferably, one of above polypeptides further comprises a sequence of an epitope from NapA, and wherein said sequence of an epitope from NapA comprises a sequence of SEQ ID NO: 28, and a sequence of SEQ ID NO: 29.

Preferably, one of above polypeptides further comprises a sequence of an epitope from BabA, and wherein said sequence of epitope from BabA comprises a sequence exhibiting at least 70%, 80%, 90% or exhibiting 100% identity to SEQ ID NO: 30. More preferably, one of above polypeptides further comprises a sequence of an epitope from BabA, and wherein said sequence of epitope from BabA comprises a sequence of SEQ ID NO: 30.

In this present invention, a polypeptide comprising two or more sequences derived from SEQ ID NO: 1-30 is defined as Multi Epitope Unit (MEU).

Preferably, one of above polypeptides comprising MEU further comprises a sequence of N-Terminus of Flagellin and/or a sequence of C-terminus of Flagellin as an adjuvant. More preferably, said sequence of N-Terminus of Flagellin comprises SEQ ID NO: 31 and/or said sequence of C-terminus of Flagellin comprises SEQ ID NO: 32, still more preferably, said SEQ ID NO: 31 and/or 32 is modified by deletion, insertion or substitution which renders the resulted sequence shows an amino acid identity of at least 70%, at least 75%, or at least 80% to the full-length of the corresponding sequence of SEQ ID NO: 31 and/or 32, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 31 and/or 32.

Preferably, one of above polypeptides comprising MEU further comprises a sequence of N-Terminus of Flagellin and a sequence of C-terminus of Flagellin as an adjuvant. More preferably, said sequence of N-Terminus of Flagellin comprises SEQ ID NO: 31 and said sequence of C-terminus of Flagellin comprises SEQ ID NO: 32, still more preferably, said SEQ ID NO: 31 and/or 32 is modified by deletion, insertion or substitution which renders the resulted sequence shows an amino acid identity of at least 70%, at least 75%, or at least 80% to the full-length of the corresponding sequence of SEQ ID NO: 31 and/or 32, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 31 and/or 32.

Preferably, one of above polypeptides comprising MEU further comprises a full-length of Flagellin as an adjuvant. More preferably, said full-length of Flagellin comprises SEQ ID NO: 33, still more preferably, said SEQ ID NO: 33 is modified by deletion, insertion or substitution which renders the resulted sequence shows an amino acid identity of at least 70%, at least 75%, or at least 80% to the full-length of the corresponding sequence of SEQ ID NO: 33, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 33.

Preferably, one of above polypeptides comprising MEU further comprises a full-length of Cholera Toxin B subunit (CTB) as an adjuvant. More preferably, said full-length of CTB comprises SEQ ID NO: 34, still more preferably, said SEQ ID NO: 34 is modified by deletion, insertion or substitution which renders the resulted sequence shows an amino acid identity of at least 70%, at least 75%, or at least 80% to the full-length of the corresponding sequence of SEQ ID NO: 34, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 34.

Preferably, one of above polypeptides comprising MEU further comprises a full-length of double mutant heat labile toxin (dmLT) as an adjuvant. More preferably, said full-length of dmLT comprises SEQ ID NO: 36, still more preferably, said SEQ ID NO: 36 is modified by deletion, insertion or substitution which renders the resulted sequence shows an amino acid identity of at least 70%, at least 75%, or at least 80% to the full-length of the corresponding sequence of SEQ ID NO: 36, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 36.

Preferably, one of above polypeptides comprises two or more of sequences selected from SEQ ID NO: 1-SEQ ID NO: 30, and wherein said two or more of sequences are linked to each other through a linker selected from a group consisting of KK, GGS, KFERQ and CTGKSC.

In this regard, KK and/or GGS can be included to avoid the generation of new epitopes. In respect of KFERQ, the lysine linker (KK) can target the lysosomal protease, cathepsin B, which is the principal enzyme for MHC-II antigen presentation, whereas KFERQ motifs can be recognized via heat shock proteins, which are important in proteasomal and lysosomal degradation pathways. With regard to CTGKSC, uptake of protein from the gut barrier can be achieved through fusion of the protein with some peptide sequences that can target specialized gut cells like M cells or Goblet cells, wherein this linker can be added to the N-terminus of the multiepitope unit to target M-cells.

Preferably, the polypeptide in the present invention is expressed by using a vaccine vector (Modified Vaccinia Ankara, MVA) carrying the coding DNA sequence of said polypeptide.

Preferably, the polypeptide in the present invention is introduced into the target cell by using a virus-like particle (VLP).

Preferably, the polypeptide in the present invention is introduced into the target cell by nanoparticles. Natural polymers such as chitosan, dextran, hyaluronic acid, cyclodextrin, alginate, and gelatin pullulan have been explored and can be used for preparation nanoparticles-based-vaccines. Even synthetic polymers can be used in vaccination of present invention. Synthetic polymers can be chitosan derivatives, poly(lactic acidglycolic acid) (PLGA), poly( -caprolactone) (PCL), dendrimers, poly(lactic acid) (PLA), poly(alkyl cyanoacrylates), polyanhydride, poly(ethylene glycol) (PEG) ,and so on which can be envisaged by a person skilled in the art.

Preferably, the polypeptide in the present invention is introduced into the target cell by combining the use of above MVA and nanoparticles.

Preferably, the polypeptide in the present invention is introduced into the target cell by combining the use of above MVA and VLP.

Preferably, the polypeptide in the present invention is introduced into the target cell by combining the use of above MVA and a mixture of polypeptide and an adjuvant as disclosed below or a fusion of polypeptide and adjuvant as disclosed above.

The sequences of SEQ ID NOs: 1-36 are laid out in the following Table 1.

**Table 1. Sequences of SEQ ID NO. 1-36**

| **SEQ ID NO.** | **Sequence** |
|---|---|
| 1 | GGGKTLREGMSQSNNP |
| 2 | IEAGAIGFKIHEDWGTTPSAINHALDVADKYDVQVAIHTDT |
| 3 | TLHDMGIFSITSSDSQAMGRVGEVITRTWQTADKNK |
| 4 | SAINHALDVADKYDVQVAIHTDT |
| 5 | QGDINELGAKFSSRDDIFSQVDT |
| 6 | PKATQDFFYGSDSKDMGGREIHQEGI |
| 7 | TGGNDPYQLMVNTKNTGEDN |
| 8 | TNGEVMGIVMKTGGNDPYQLMVNTKNTGEDN |
| 9 | TLLSSLKGPV |
| 10 | SALNSNPKATQDFFYGSDSKDMGGREIHQEG |
| 11 | TLLSSLKGPVK |
| 12 | RSSLNNVFSYSVHTDNGVESLMKYGLSL |
| 13 | TLLSSLKGPV |
| 14 | GEVMGIVMKTGGNDP |
| 15 | DDIFSQVDT |
| 16 | DPKRTIQKKSEPGLLFSTGLDKMEG |
| 17 | EPMSGESLDSFTMDLSELDIQEKFLKTTHSSHSGGLVSTMVKGTDNSND |
| 18 | KVTILEPMSGESLDSF |
| 19 | VLIPAGFIKVTI |
| 20 | GESLDSFTMDLSELDIQEKFLK |
| 21 | LTQKNLESYQKDAKELQNKR |
| 22 | ALKVEQILQNQGYKVISVDS |
| 23 | SANDKRMQDNLVSVIEKQTNKKVRILEIKPLKSS |
| 24 | SVIEKQTNKKVRILEIKPLKSSQD |
| 25 | TNKKVRILEIKPLKSSQ |
| 26 | VWGWLGVNSAKKAALI |
| 27 | EEMAKARARGASVEDKISILEKIYSTQYDINAQKEPED |
| 28 | QLGHHPLVTLSEA |
| 29 | TRVKEETKTSF |
| 30 | SAINTNEQSTPIGESGKNFNPFKDAS |
| 31 | MAQVINTNSLSLLTQNNLNKSQSSLSSAIERLSSGLRINSAKDDAAGQAIANRFTSNIKGLT QASRNANDGISIAQTTEGALNEINNNLQRVRELSVQATNGTNSDSDLKSIQDEIQQRLEEI DRVSNQTQFNGVKVLSQDN |
| 32 | LASIDSALSKVDAVRSSLGAIQNRFDSAITNLGNTVTNLNSVRSRIEDADYATEVSNMSKA QILQQAGTSVLAQANQVPQNVLSLLR |
| 33 | MAQVINTNSLSLLTQNNLNKSQSSLSSAIERLSSGLRINSAKDDAAGQAIANRFTSNIKGLT QASRNANDGISIAQTTEGALNEINNNLQRVRELSVQATNGTNSDSDLKSIQDEIQQRLEEI DRVSNQTQFNGVKVLSQDNQMKIQVGANDGETITIDLQKIDVKSLGLDGFNVNGPKEAT VGDLKSSFKNVTGYDTYAAGADKYRVDINSGAVVTDAAAPDKVYVNAANGQLTTDDAE NNTAVNLFKTTKSTAGTDEAKAIAGAIKGGKEGDTFDYKGVSFTIDTKAGNDGNGTVSTTI NGEKVTLTVADITAGAANVNDATLQSSKNVYTSVVNGQFTFDDKTKNESAKLSDLEANNA VKGESKITVNGAEYTANAAGDKVTLAGKTMFIDKTASGVSTLINEDAAAAKKSTANPLASID |
| | SALSKVDAVRSSLGAIQNRFDSAITNLGNTVTNLNSVRSRIEDADYATEVSNMSKAQILQQ AGTSVLAQANQVPQNVLSLLR |
| 34 | MTPQNITDLCAEYHNTQIYTLNDKIFSYTESLAGKREMAIITFKNGAIFQVEVPGSQHIDSQK KAIERMKDTLRIAYLTEAKVEKLCVWNNKTPHAIAAISMANGT |
| 35 | SITCSFNNLGIGYFGVMSIENMKKLNEAYQILQAALKQGLPALKENNGTIKDVTYSYICY GEGNNNCEIKLADGQKLQQNGGSETTTQTIDGKTVSTTISSKVVDSTAADNTYKQSYTEI TNALKDVPDNAQALLAQASTLINTINTACPYFSVTNSSSPNAPKMEPTKGKLCGFTDEIS AIQKMITDAQELVNQTSAINTNEQSTPIGESGKNFNPFKDASFAQGMLANASAQAKMLSL ADQVGQAINPDRLTGN |
| 36 | CAT ATG ATG GGA GAT CGC CTG TAT CGT GCT GAT AGC CGT CCA CCG GAC GAA ATC AAA CGC TCG GGT GGC TTA ATG CCG CGT GGT CAT AAC GAG TAC TTT GAT CGT GGT ACG CAG ATG AAC ATC AAT CTG TAT GAC CAC GCA CGT GGC ACA CAA ACT GGC TTC GTT CGG TAT GAC GAT GGC TAT GTC AGC ACT TCG CTC TCG TTA CGT TCT GCC CAC TTA GCC GGC CAA AGC ATT CTG AGC GGG TAT AGC ACG TAT TAC ATC TAC GTG ATC GCT ACT GCA CCG AAT ATG TTC AAC GTG AAT GAC GTG TTG GGC GTT TAC AGT CCA CAT CCG TAC GAA CAG GAA GTT AGT GCG CTG GGA GGC ATT CCG TAC TCC CAG ATC TAT GGG TGG TAT CGC GTC AAC TTT GGG GTG ATC GAT GAA CGG CTT CAT CGC AAT CGC GAG TAT CGT GAT CGT TAC TAC CGC AAC CTG AAC ATT GCC CCA GCG GAA GAT GGT TAC CGC CTT GCC GGC TTT CCG CCG GAT CAT CAG GCA TGG CGC GAA GAA CCG TGG ATT CAC CAT GCT CCC CAA GGC TGT GGC AAT AGC AGT GGT ACC ATT ACC GGG GAC ACG TGC AAC GAA GAA ACG CAA AAT CTG TCG ACA ATT TAC GCC CGT GAG TAT CAG TCA AAA GTC AAA CGC CAA ATT TTC TCC GAT TAT CAG TCT GAA GTA GAC ATC TAC AAT CGC ATT CGC GAT GAA CTC GGC GGT TCA GGT GGA TCA GGT GGG AGT GCG CCT CAG ACC ATT ACC GAG CTG TGT TCC GAG TAT CGC AAT ACG CAG ATT TAT ACG ATC AAC GAC AAA ATT CTG TCG TAT ACT GAG AGC ATG GCG GGT AAA CGC GAA ATG GTG ATT ATC ACC TTT AAA AGC GGT GAA ACC TTT CAG GTT GAG GTA CCT GGC TCT CAA CAC ATC GAT TCA CAG AAG AAA GCG ATT GAA CGT ATG AAA GAT ACC CTG CGC ATT ACC TAT CTG ACC GAA ACC AAG ATC GAT AAA TTG TGC GTG TGG AAC AAC AAA ACA CCC AAC TCT ATT GCA GCG ATT TCC ATG AAG AAT CTC GAG |

In some embodiments, one or more of above SEQ ID NOs: 1-30 may be modified by deletion, insertion or substitution which renders the resulted sequence shows an amino acid identity of more than 70%, preferably more than 75%, more preferably more than 80% to the full-length of the corresponding sequence of SEQ ID NO. 1-30, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO. 1-30.

Preferably, one of above polypeptides is for use preventing or treating an infection by *H. pylori* and/or a disease caused by *H. pylori* infection. Said disease caused by *H. pylori* infection can be gastritis (in all patients infected, see Blaser MJ. Hypothesis: the changing relationships of Helicobacter pylori and humans: implications for health and disease. J Infect Dis. 1999; 179:1523-1530), gastric and duodenal ulcers and gastric carcinoma. In patients suffering from gastritis, there might be swelling or redness in the lining of stomach, and patient might have peptic ulcers or sores in small intestine or stomach. The patients suffering from ulcer might have abdominal pain or belly pain, wherein the patients may have a dull pain which occur after meal or in the middle of night, and said pain may occur in two or three hours after meal and may disappear after the patient takes medicine which can reduce the stomach acid level.

Preferably, in above polypeptide for use, said polypeptide is used as a therapeutic and/or a prophylactic vaccine against an infection by Helicobacter pylori (*H. pylori)* and/or a disease caused by *H. pylori* infection.

Preferably, in any of above polypeptide for use, a separate polypeptide comprising a sequence of N-Terminus of Flagellin and/or a sequence of C-terminus of Flagellin is used as an adjuvant to mix with above polypeptide for use. More preferably said sequence of N-Terminus of Flagellin comprises SEQ ID NO: 31 and/or said sequence of C-terminus of Flagellin comprises SEQ ID NO: 32, still more preferably, said SEQ ID NO: 31 and/or said SEQ ID NO: 32 is modified by deletion, insertion or substitution which renders the resulted sequence shows an amino acid identity of at least 70%, at least 75%, or at least 80% to the full-length of the corresponding sequence of SEQ ID NO: 31 and/or 32, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 31 and/or 32.

Preferably, in any of above polypeptide for use, a separate polypeptide comprising a sequence of N-Terminus of Flagellin and a sequence of C-terminus of Flagellin is used as an adjuvant to mix with above polypeptide for use. More preferably said sequence of N-Terminus of Flagellin comprises SEQ ID NO: 31 and said sequence of C-terminus of Flagellin comprises SEQ ID NO: 32, still more preferably, said SEQ ID NO: 31 and/or said SEQ ID NO: 32 is modified by deletion, insertion or substitution which renders the resulted sequence shows an amino acid identity of at least 70%, at least 75%, or at least 80% to the full-length of the corresponding sequence of SEQ ID NO: 31 and/or 32, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 31 and/or 32.

Preferably, in any of above polypeptide for use, a separate polypeptide comprising or being a sequence of full-length Flagellin is used as an adjuvant to mix with above polypeptide for use. More preferably said full-length Flagellin comprises SEQ ID NO: 33, still more preferably, said SEQ ID NO: 33 is modified by deletion, insertion or substitution which renders the resulted sequence displaying an amino acid identity of at least 70%, at least 75%, or at least 80% to the corresponding sequence of SEQ ID NO: 33, or shows an amino acid identity of 80%, 81%, 82%, 83%, 84%, 85%, 86%, 87%, 88%, 89%, 90%, 91%, 92%, 93%, 94%, 95%, 96%, 97%, 98%, 99% or 100% to the corresponding sequence of SEQ ID NO: 33.

Preferably, in any of above polypeptide for use, a separate polypeptide comprising or being a sequence of full-length CTB is used as an adjuvant to mix with above polypeptide for use. More preferably said full-length CTB comprises SEQ ID NO: 34, still more preferably, said SEQ ID NO: 34 is modified by deletion, insertion or substitution which renders the resulted sequence displaying an amino acid identity of at least 70%, at least 75%, or at least 80% to the corresponding sequence of SEQ ID NO: 34, or shows an amino acid identity of 80%, 81%, 82%, 83%, 84%, 85%, 86%, 87%, 88%, 89%, 90%, 91%, 92%, 93%, 94%, 95%, 96%, 97%, 98%, 99% or 100% to the corresponding sequence of SEQ ID NO: 34.

Preferably, in any of above polypeptide for use, a separate polypeptide comprising or being a sequence of full-length dmLT is used as an adjuvant to mix with above polypeptide for use. More preferably said full-length dmLT comprises SEQ ID NO: 36, still more preferably, said SEQ ID NO: 36 is modified by deletion, insertion or substitution which renders the resulted sequence displaying an amino acid identity of at least 70%, at least 75%, or at least 80% to the corresponding sequence of SEQ ID NO: 36, or shows an amino acid identity of 80%, 81%, 82%, 83%, 84%, 85%, 86%, 87%, 88%, 89%, 90%, 91%, 92%, 93%, 94%, 95%, 96%, 97%, 98%, 99% or 100% to the corresponding sequence of SEQ ID NO: 36.

Preferably, the amount of any of above disclosed adjuvant to the amount of above disclosed polypeptide for use is in a ratio of 1: 1 to 1: 20, 1: 1 to 1: 15, 1: 2 to 1: 20, 1: 2 to 1: 15, 1 : 2 to 1: 10, 1 : 3 to 1: 9, 1 : 4 to 1: 8, 1 : 5 to 1: 7, 1: 3 to 1: 8 or 1: 3 to 1 : 6.

Preferably, in above polypeptide for use, said polypeptide is administered into a subject in need thereof in a prophylactic or therapeutically effective amount.

In some embodiments, a prophylactic or therapeutically effective amount of the abovedisclosed polypeptide may be administered orally or non-orally e.g. intramuscular injection. Oral administration includes but is not limited to: topical administration, or parenteral administration, or combinations thereof. Administration by injection includes but is not limited to: via the intramuscular or intraperitoneal route, or the composition of the present invention can be injected directly into the target tumor. A "subject", "individual" or "patient" may be a vertebrate, such as a human. The vertebrate may be a mammal.

Preferably, in above polypeptide for use, said polypeptide is administered into the subject together with pharmaceutically acceptable carriers and/or excipients including but not being limited to: stabilizing agents, surface-active agents, salts, buffers, colouring agents etc.

The present application also provides a composition, preferably a pharmaceutical composition, comprising one or more of above polypeptides.

Preferably, said composition comprises pharmaceutically acceptable carriers and/or excipients including but not being limited to: stabilizing agents, surface-active agents, salts, buffers, colouring agents etc.

Preferably, one of above composition further comprises a separate polypeptide comprising a sequence of N-Terminus of Flagellin and/or a sequence of C-terminus of Flagellin to be used as an adjuvant. More preferably, said N-Terminus of Flagellin comprises SEQ ID NO: 31 and/or said C-terminus of Flagellin comprises SEQ ID NO: 32, still more preferably said SEQ ID NO: 31 and/or 32 is modified by deletion, insertion or substitution which renders the resulted sequence shows an amino acid identity of at least 70%, at least 75%, or at least 80% to the full-length of the corresponding sequence of SEQ ID NO: 31 and/or 32, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 31 and/or 32. Preferably, one of above composition further comprises a separate polypeptide comprising a sequence of N-Terminus of Flagellin and a sequence of C-terminus of Flagellin to be used as an adjuvant. More preferably, said N-Terminus of Flagellin comprises SEQ ID NO: 31 and said C-terminus of Flagellin comprises SEQ ID NO: 32, still more preferably said SEQ ID NO: 31 and/or 32 is modified by deletion, insertion or substitution which renders the resulted sequence shows an amino acid identity of at least 70%, at least 75%, or at least 80% to the full-length of the corresponding sequence of SEQ ID NO: 31 and/or 32, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 31 and/or 32.

Preferably, one of above composition further comprises a separate polypeptide comprising a sequence of full-length Flagellin to be used as an adjuvant. More preferably, said full-length Flagellin comprises SEQ ID NO: 33, still more preferably said SEQ ID NO: 33 is modified by deletion, insertion or substitution which renders the resulted sequence shows an amino acid identity of at least 70%, at least 75%, or at least 80% to the full-length of the corresponding sequence of SEQ ID NO: 33, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 33.

Preferably, one of above composition further comprises a separate polypeptide comprising a sequence of full-length Cholera Toxin B subunit (CTB) to be used as an adjuvant. More preferably, said full-length CTB comprises SEQ ID NO: 34, still more preferably said SEQ ID NO: 34 is modified by deletion, insertion or substitution which renders the resulted sequence shows an amino acid identity of at least 70%, at least 75%, or at least 80% to the full-length of the corresponding sequence of SEQ ID NO: 34, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 34.

Preferably, one of above composition further comprises a separate polypeptide comprising a sequence of full-length double mutant heat labile toxin (dmLT) to be used as an adjuvant. More preferably, said full-length dmLT comprises SEQ ID NO: 36, still more preferably said SEQ ID NO: 36 is modified by deletion, insertion or substitution which renders the resulted sequence shows an amino acid identity of at least 70%, at least 75%, or at least 80% to the full-length of the corresponding sequence of SEQ ID NO: 36, or shows an amino acid identity of 80%, 81%, 82%, 83%, 84%, 85%, 86%, 87%, 88%, 89%, 90%, 91%, 92%, 93%, 94%, 95%, 96%, 97%, 98%, or 99% to the corresponding sequence of SEQ ID NO: 36.

Preferably, above separate polypeptide as adjuvant in the composition is mixed with other polypeptides disclosed in the present invention, said other polypeptides can be other adjuvants and/or MEU.

More preferably, above composition in the present invention is in the form of a kit of parts.

Preferably, one of above composition is for use preventing or treating of infection by *H. pylori* and/or a disease caused by *H. pylori* infection. Said disease caused by *H. pylori* infection can be gastritis (in all patients infected, see Blaser MJ. Hypothesis: the changing relationships of Helicobacter pylori and humans: implications for health and disease. J Infect Dis. 1999;179:1523-1530), gastric and duodenal ulcers and gastric carcinoma. In patients suffering from gastritis, there might be swelling or redness in the lining of stomach, and patient might have peptic ulcers or sores in small intestine or stomach. The patients suffering from ulcer might have abdominal pain or belly pain, wherein the patients may have a dull pain which occur after meal or in the middle of night, and said pain may occur in two or three hours after meal and may disappear after the patient takes medicine which can reduce the stomach acid level.

Preferably, in above composition for use, said composition is used as a vaccine against *H. pylori* infection and/or disease caused by *H. pylori* infection.

Preferably, in above composition for use, said composition is administered into a subject in need thereof in a prophylactic or therapeutically effective amount.

In some embodiments, a prophylactic or therapeutically effective amount of the abovedisclosed composition may be administered orally or non-orally e.g. intramuscular injection. Oral administration includes but is not limited to: topical administration, sublingual administration, nasal spray, parenteral administration, or combinations of any two or all four of them. Administration by injection includes but is not limited to: via the intramuscular or intraperitoneal route, or the composition of the present invention can be injected directly into the target tumor. A "subject", "individual" or "patient" may be a vertebrate, such as a human. The vertebrate may be a mammal.

Preferably, any of above polypeptides or compositions is for use in treating tumor, cancer or metastasis. More preferably, said tumor, cancer or metastasis is caused by *H. pylori* infection.

In the present application, the terms "treatment", "treat" and "treating" refer to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The peptide or the composition of the present invention may be used to delay development of a disease or disorder or to slow the progression of a disease or disorder. The term "treating", "treatment" or "alleviation" refers to improving, alleviating, and/or decreasing the severity of one or more symptoms of a condition being treated. For example, treating cancer refers to improving the patient's condition, alleviating, delaying or slowing progression or onset, decreasing the severity of one or more symptoms of cancer. For example, treating cancer includes any one or more of: decreasing tumor size, decreasing rate of tumor size increase, halting increase in size, decreasing the number of metastases, decreasing pain, increasing survival, and increasing progression free survival.

A "therapeutically effective amount" of the polypeptide or the composition in the present invention is variable according to factors such as age, sex, the disease state, weight of the subject, and the ability of the substance/molecule, to elicit a desired response in the subject. A therapeutically effective amount may encompass an amount in which any toxic or detrimental effects of the composition are outweighed by the therapeutically beneficial effects. Preferably, said prophylactic or therapeutically effective amount of the polypeptide derived from SEQ ID NO: 1-30 as disclosed above or said therapeutically effective amount of MEU is 5-100 µg per subject, 10-90 µg per subject, 20-80 µg per subject, 30-70 µg per subject, 40-60 µg per subject, 20-100 µg per subject, 30-100 µg per subject, 30-90 µg per subject, 30-80 µg per subject, 30-70 µg per subject, 30-60 µg per subject, or 30-50 µg per subject. More preferably, said therapeutically effective amount of the polypeptide is 20-100 µg per subject, 30-100 µg per subject, or 30-90 µg per subject.

Preferably, the polypeptide comprising MEU is expressed by using a vaccine vector MVA (i.e. MVA-MEU).

Preferably, in MVA-MEU comprises the full-length of BabA, more preferably said full-length of BabA comprises or is SEQ ID NO: 30 or 35, still more preferably, said SEQ ID NO: 30 or 35 is modified by deletion, insertion or substitution which renders the resulted sequence displaying an amino acid identity of at least 70%, at least 75%, at least 80% to the corresponding sequence of SEQ ID NO: 30 or 35, or shows an amino acid identity of 80%, 81%, 82%, 83%, 84%, 85%, 86%, 87%, 88%, 89%, 90%, 91%, 92%, 93%, 94%, 95%, 96%, 97%, 98%, 99% or 100% to the corresponding sequence of SEQ ID NO: 30 or 35.

Preferably, the prophylactic or therapeutically effective amount of MVA-MEU is 10⁶ to 10⁸ PFU, or 10⁶ to 10⁷ PFU per subject. More preferably, the therapeutically effective amount of MVA-MEU is 2 × 10⁷ to 8 × 10⁷ PFU, 3 × 10⁷ to 7 × 10⁷ PFU, or 4 × 10⁷ to 6 × 10⁷ PFU per subject.

Preferably, above disclosed adjuvant is administered in amount of 0.5-20 µg per subject, 1-19 µg per subject, 2-18 µg per subject, 3-17 µg per subject, 4-16 µg per subject, 5-15 µg per subject, 6-14 µg per subject, 7-13 µg per subject, 8-12 µg per subject, 3-15 µg per subject, 4-12 µg per subject, or 5-10 µg per subject. More preferably, above disclosed adjuvant is administered in amount of 0.5-10 µg per subject, 1-10 µg per subject, 1.5-10 µg per subject, or 2-10 µg per subject.

Preferably, said therapeutically effective amount of the composition is 0.5-5 µg per subject, 1-4.5 µg per subject, 1.5-4 µg per subject, 2-3.5 µg per subject, 2.5-3 µg per subject, 1-4 µg per subject, 2-3 µg per subject, 1-3.5 µg per subject, 1-3 µg per subject, 1-2.5 µg per subject, 1-2 µg per subject, 1-1.5 µg per subject More preferably, said therapeutically effective amount of the composition is 1-3.5 µg per subject, 1-2.5 µg per subject, or 1-1.5 µg per subject.

Preferably, the polypeptide or the composition is administered to one subject in a total number of 2 times, 3 times, 4 times, 5 times, 6 times, 7 times, 8 times, 9 times, 10 times, 11 times, 12 times, 13 times, 14 times, 15 times..

Preferably, the interval of two administration of polypeptide or composition is one week, two weeks, three weeks, four weeks, or five weeks. More preferably, if the polypeptide or composition is administered into human, the interval of two administration is two weeks, three weeks, or four weeks.

Preferably, three sequential administration have two different intervals, wherein the difference between said two intervals is one week, two weeks, three weeks or four weeks.

Preferably, the last interval is four weeks, and the rest of intervals are all one week.

Preferably, if the polypeptide or composition is administered into human, the last interval is 3 months, 2 months, 1 month, 4 weeks, 3 weeks, or 2 weeks.

Preferably, one week interval in mouse study can be translated as 3-4 weeks for human.

Preferably, the polypeptide or the composition is administered for one treatment cycle, two treatment cycles, three treatment cycles, four treatment cycles, or five treatment cycles.

Preferably, one treatment cycle means that the polypeptide or the composition is administered for 2 times, 3 times, 4 times, or 5 times.

Preferably, one treatment cycle means that the polypeptide or the composition is administered for 4 times, wherein the administration for the first 3 times is performed with one week interval, and the administration for the 4^{th} time is performed with an interval of four weeks.

In this regard, one week in mouse study as disclosed above is usually corresponding to 1 month in human clinical trials.

### Figures

Fig.1: Immune cell response of each immunised animal group.
Fig.2: Amount of antibodies in serum of immunised animals.
Fig.3: Anti-MEU antibody response in immunised animal groups.
Fig.4: Anti-MEU antibodies can bind to the *H. pylori's* natural crude lysate.
Fig.5: Reactivity of the human sera with MEU.
Fig.6: Bacterial load in different animal groups.
Fig.7: Bacterial plate of animal group 8 compared to the control group.
Fig.8: Immune cell response of each animal group.
Fig.9: level of MEU specific antibodies in the serum of the immunised mice.
Fig.10: Avidity of anti-MEU in animal groups 8 and 9.
Fig. 11: Increase of immune CD4⁺CD8⁺NK1.1⁺ and CD4⁻CD8⁻NK1.1⁺ cells after immunisation.

### Examples

The present invention will now be described in detail with reference to examples thereof. However, these examples are illustrative and do not limit the scope of the invention.

### Example 1 Production of the recombinant proteins

The final designed constructs (MEU, Chimeric flagellin-MEU and chimeric dmLT-MEU were analysed from different point of views like antigenicity, solubility, allergenicity, physicochemical properties and 3D structural modeling. For this purpose, a variety of online available tools like Soluprot, Vaxigen2, I-Tasser, COACH-D and AllergenFP were applied. The MEU (Multi Epitope Unit) antigen consists of 16 epitopes (SEQ ID NO: 1, 2, 3, 5, 8, 10, 11, 16, 17, 19, 22, 23, 24, 26, 29, 30) from UreaseB, HpaA, Hp0231, NapA and BabA. Chimeric flagellin-MEU is directed to a fusion polypeptide of MEU and N-terminus (SEQ ID NO: 31), C-terminus (SEQ ID NO: 32) or full-length (SEQ ID NO: 33) of Flagellin. Chimeric dmLT-MEU is directed to a fusion polypeptide of MEU and full-length dmLT (SEQ ID NO: 36).

The designed proteins were inserted in the pET28a+ expression vector and cloned in E-coli. The expression protocol is as followed.

Briefly, the coding sequence of the designed protein was optimized for the expression in E.coli and synthetized. The ORF (open reading frame) of the protein was cloned into the plasmid pET28a. The E.coli bacteria (strain BL21) were transformed with the modified plasmid and gene expression was induced using IPTG. Expression was done overnight at 18°C in the shaking LB medium. The next day, cells were harvested, and the cell pellet was resuspended in lysis buffer and lysed sonication. Soluble lysate was incubated with Ni-NTA resins at 6-8°C for 1 h. The mixture was applied on the IMAC chromatography column. Column as washed and protein was eluted subsequently. IMAC eluted fractions were collected. After the Tag cleavage using the thrombin protease, the protein was applied on the size exclusion chromatography (SEC) column HiLoad Superdex. The SEC eluates of the expected fractions (based on the product size) were collected and stored at -70°C until use for immunisation.

### Example 2 Immunisation of animals

The MEU (Multi Epitope Unit) antigen consists of 16 epitopes (SEQ ID NO: 1, 2, 3, 5, 8, 10, 11, 16, 17, 19, 22, 23, 24, 26, 29, 30) from UreaseB, HpaA, Hp0231, NapA and BabA. Compared to

MEU, the MVA-MEU version (expression of epitopes via MVA) has the full-length protein of BabA with a sequence of SEQ ID NO: 35.

The aim of this part of the animal study was mainly to evaluate the immunogenicity of the new vaccine constructs (MEU + separate adjuvant or MVA-MEU). Moreover, different roots of administration have been tested. Apart from immunogenicity, the overall health condition of the animals was monitored.

The following groups of mice have been included in the study. Each group 6 female 6-8 weeks old C57/BI6 mice. Treatments with 1 week interval, end analysis 2 weeks after the 3^{rd} immunisation.

**Table 2. Immunisation study**

| **Treatment** | **C57BL/6J** | **Group** |
|---|---|---|
| Immunisation; Flagellin+MEU: intramuscular (i.m.), i.m., i.m. | 6 | 1 |
| 5µg Flagellin + 30µg MEU | | |
| Immunisation; CTB+MEU: i.m., i.m., i.m. | 6 | 2 |
| 10 µg CTB + 30µg MEU | | |
| Immunisation; CTB+MEU: oral, i.m., i.m. | 6 | 3 |
| Oral: 30 µg CTB + 30µg MEU | | |
| IM: 10 µg CTB + 150µg MEU | | |
| Immunisation; CTB+MEU: oral, oral, and MVA-MEU: i.m. | 6 | 4 |
| 3×10⁷/mouse | | |
| Immunisation; Flagellin+MEU: i.m., i.m., and MVA-MEU: i.m. | 6 | 5 |
| Immunisation; CTB+MEU: i.m., i.m., and MVA-MEU: i.m. | 6 | 6 |
| Control group | 6 | 7 |
| **Sum** | **42** | |

Apart from the serum which was collected before each treatment, the spleen and a piece of the stomach was also collected and subjected in the analysis.

### Example 3 Cellular immune response

It is of importance that the immunisation induces not only a good humoral response (antibody) but a significant cellular immune response. This point has been analysed in the cells isolated from the spleen of the animals followed by 24 hours stimulation with the antigen (MEU) compared to positive control.

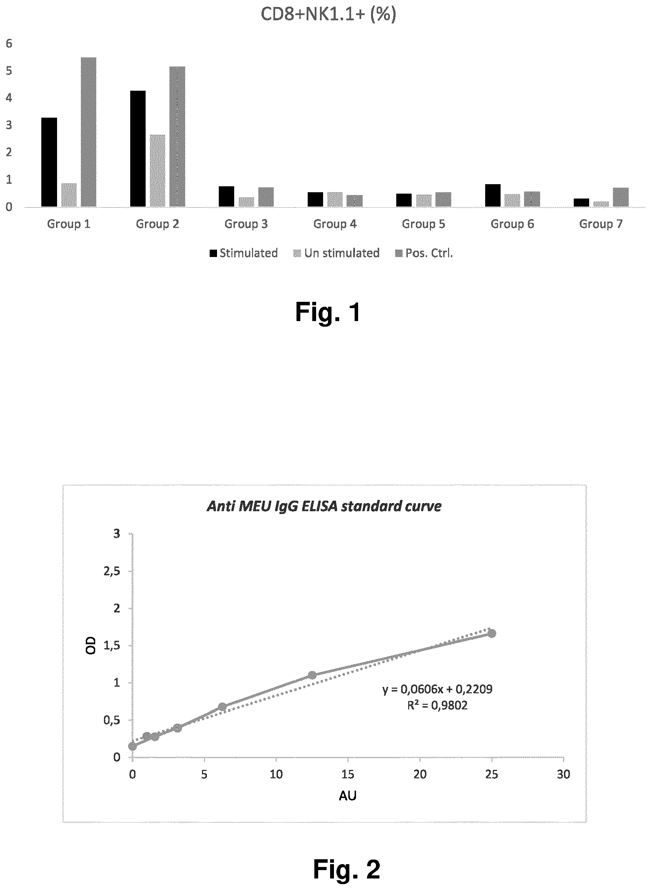

As showed in Fig. 1, immune cell response of each group is depicted. It could be concluded that the new MEU antigen especially in combination with flagellin or CTB as adjuvant is able to induce NKT-cells. These cells play a crucial role in immune response against *H. pylori.* A reasonable NKT cell immune response could be observed in the immunised animals.

### Example 4 Humoral immune response

To evaluate the humoral immune response, the serum of the animals was collected, and antibodies (IgG) was analysed in an ELISA which is developed exclusively for this study.

Fig. 2 shows the standard curve of the developed ELISA. The identified lower limit of detection (LLoD) of this ELISA is 1.03 AU/ml.

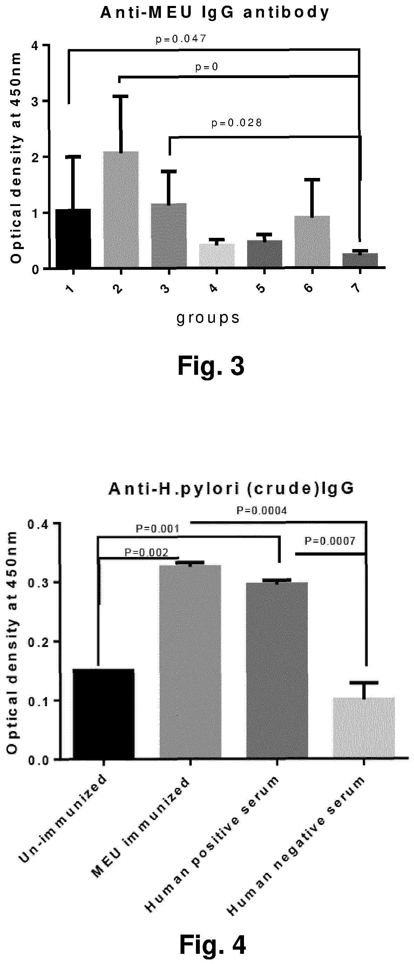

Using this ELISA, the mouse sera were analysed. As showed in Fig. 3, the anti-MEU antibody response in different groups is demonstrated. It is concluded that the MEU is an antigen which in combination with flagellin or CTB as adjuvant induces antibody response.

The anti-MEU antibodies can bind to the *H. pylori's* natural crude lysate. This level of antibody is comparable to the antibody response in the serum of *H. pylori* infected human. This demonstrates the correct antigen processing and presenting of the epitopes of MEU in the same way as how they are presented in the natural antigen processing of the intact helicobacter's antigens. The Fig. 4 shows the above finding.

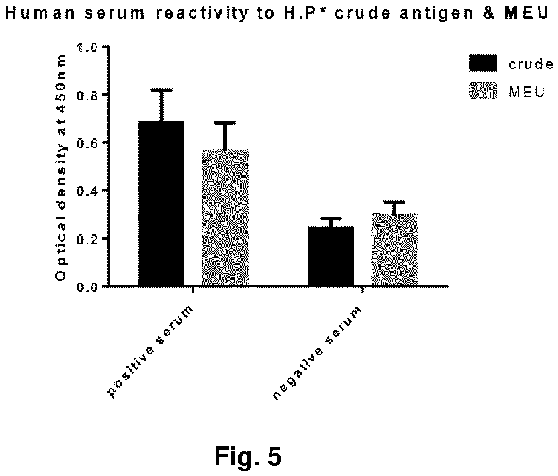

Moreover, to observe the reactivity of the human *H. pylori's* positive sera with the new synthetic MEU, human sera were analysed in the ELISA, parallel to the animal studies. Briefly, the antigen MEU was coated in the ELISA plates and presence of antibodies against it and/or its epitopes in the human sera was detected. Fig. 5 shows the presence and reactivity of the human sera with our antigen. This confirms the correctness of the epitopes selected in designing of the new vaccine.

### Example 5: Efficacy study by using animal model

In order to evaluate the efficacy of the immune response upon the immunisation with the new vaccine variants, groups of each 6 female 6-8 weeks old C57/BI6 mice were immunised 4 weeks after the infection of mice. These groups were compared to the control groups of nonimmunised or un-infected mice. The table below show the details of treated animal groups. Mice were infected with 2×10⁸ freshly cultured *H. pylori* strain SS1 via oral gavage. They received 3 doses of bacteria orally every second day. Immunisation was started 4 weeks after the 3^{rd} infection. Mice were immunised with 1-week intervals according to the schedule depicted in the table below. Blood samples were collected prior to each immunisation doses. The animals were sacrificed for the final analysis 4 weeks after the last immunisation dose.

**Table 3. Efficacy study**

| **Treatment** | **C57BL/6J** | **Group** |
|---|---|---|
| Control group - Infected, **not** immunised | 6 | 1 |
| Adj. ctrl. group - Infected, immunised with Flagellin: i.m., i.m., i.m. | 5 | 2 |
| Adj. ctrl. group - Infected, immunised with CTB: i.m., i.m., i.m. | 5 | 3 |
| Infection+Immunisation; Flagellin+separate MEU: i.m., i.m., i.m. | 6 | 4 |
| 5µg Flagellin + 30µg MEU | | |
| Infection+Immunisation; CTB+separate MEU: i.m., i.m., i.m. | 6 | 5 |
| 10 µg CTB + 30µg MEU | | |
| Infection+Immunisation; CTB+separate MEU: oral, i.m., i.m. | 6 | 6 |
| Oral: 30 µg CTB + 150µg MEU | | |
| IM: 10 µg CTB + 30µg MEU | | |
| Infection+Immunisation; CTB+separate MEU: oral, oral, and MVA-MEU: i.m. | 6 | 7 |
| 3×10⁷/mouse | | |
| Infection+Immunisation; Flagellin+separate MEU: i.m., i.m., MVA-MEU: i.m. | 6 | 8 |
| Infection+Immunisation; CTB+separate MEU: i.m., i.m., MVA-MEU: i.m. | 6 | 9 |
| Control group - **not** Infected, **not** immunised | 5 | 10 |
| **Sum** | **57** | |

### Example 6: Bacterial load in the gastric mucosa

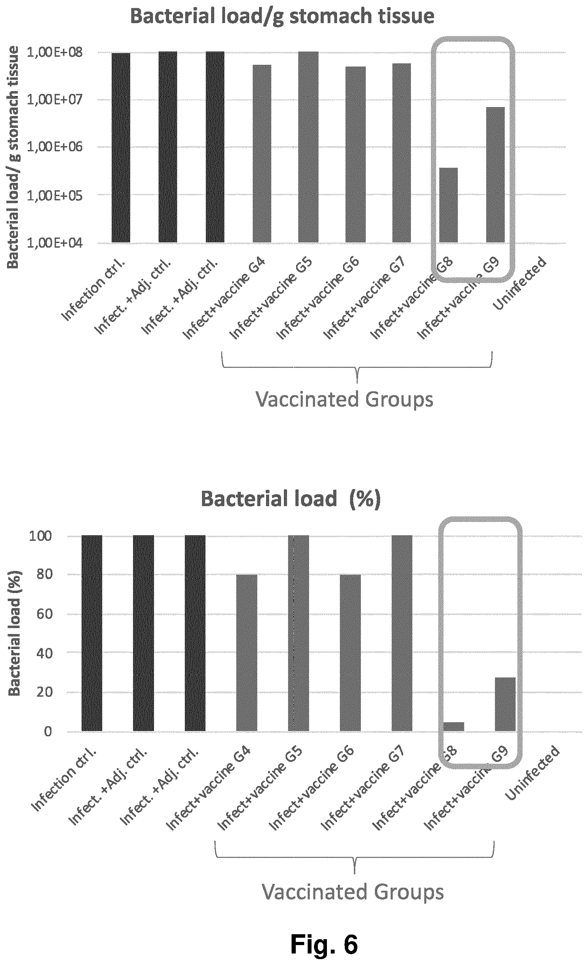

To analyse the bacterial load in the stomach of the animals, this organ was taken from the animals and a part of it was homogenised and used for bacterial culture. Briefly, around 1/3 of the stomach was homogenised in the BHI medium supplemented by 10% FBS and serial dilutions of the suspension were seeded on the Columbia blood agar plates supplemented by 10% FCS. Typical *H. pylori's* colonies were observed and counted 36 hours after the plating. Fig. 6 shows a significant reduction of the bacterial load in the mice of group 8 and 9. The bacterial load is normalized to the weight of the input tissue.

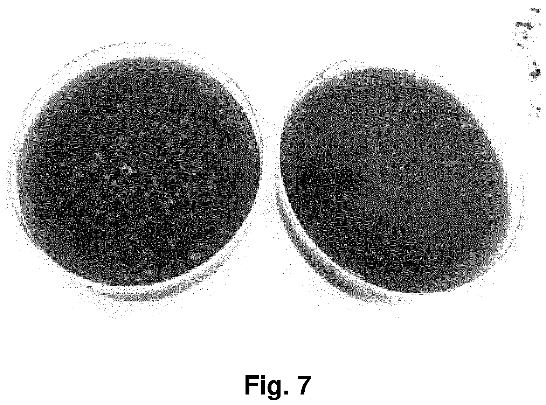

Particularly, Fig. 7 clearly shows the bacterial plate of group 8 compared to the control group.

### Example 7: Cellular immune response:

Cellular immune response has been analysed in the cells isolated from the spleen of the animals followed by 24 hours stimulation with the antigen (MEU) compared to PMA/lonomycin as positive control.

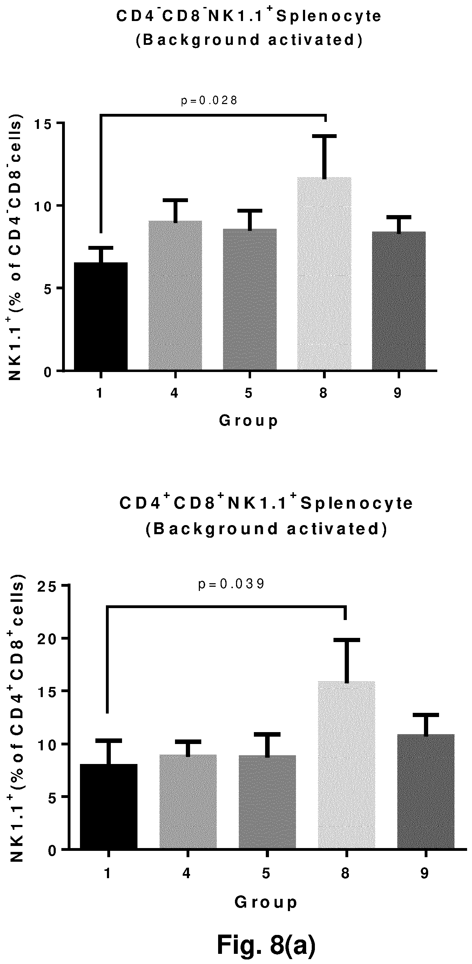

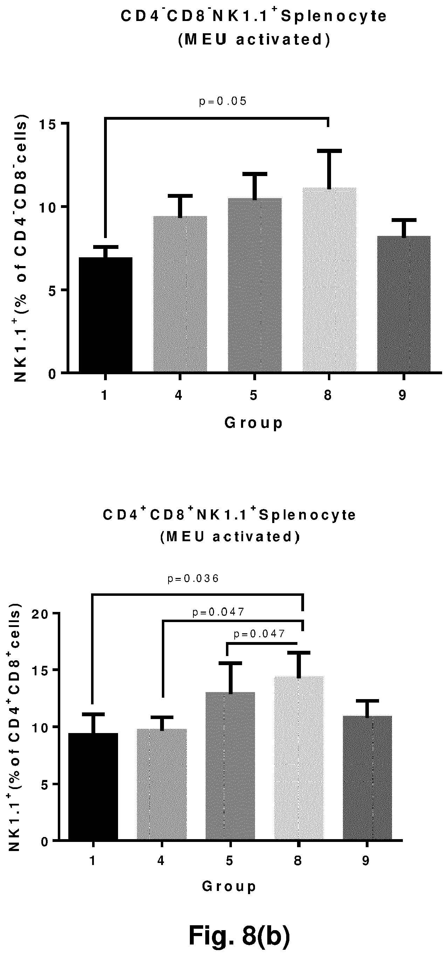

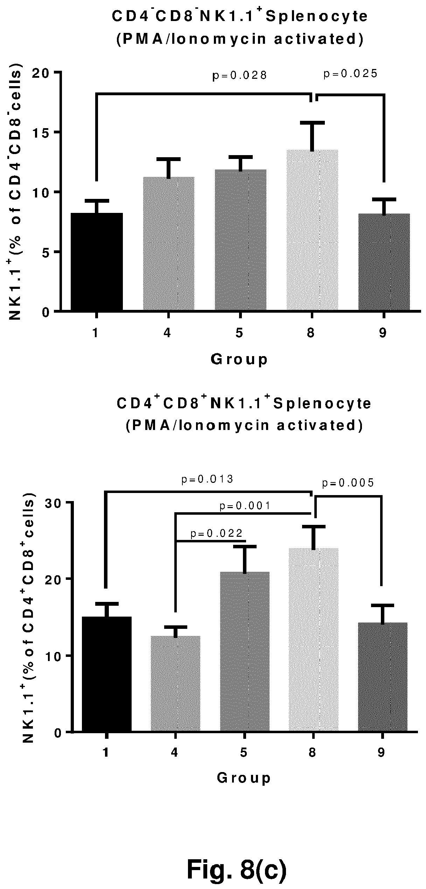

In Fig. 8(a)-(c), immune cell response of each group is depicted. It is shown that the MEU antigen especially in combination with flagellin or CTB as adjuvant is able to induce NKT-cells. These cells play a crucial role in immune response against *H. pylori.* More specifically, subsequent to the stimulation, cells were stained with specific antibodies against CD4, CD8, CD25, CD107a and NK1.1.

Moreover, the cytokines IL2, IL4, IL17 and IFN-γ were analysed in the supernatant of the splenocytes after the stimulation. The graphics below show the level of these cytokines.

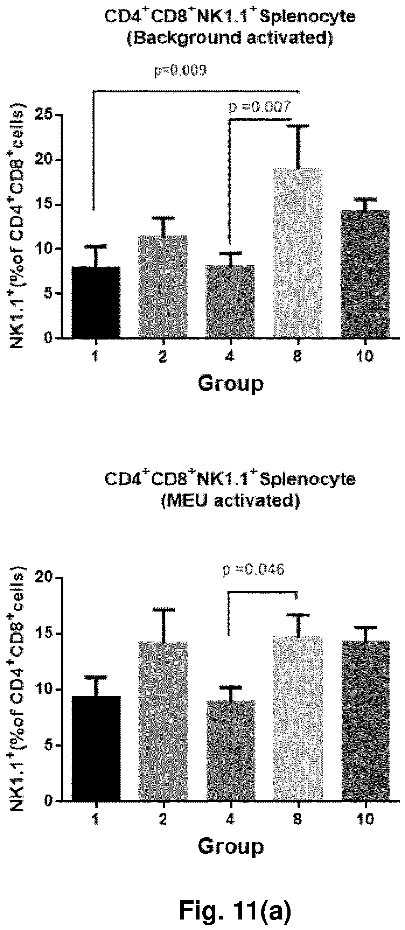

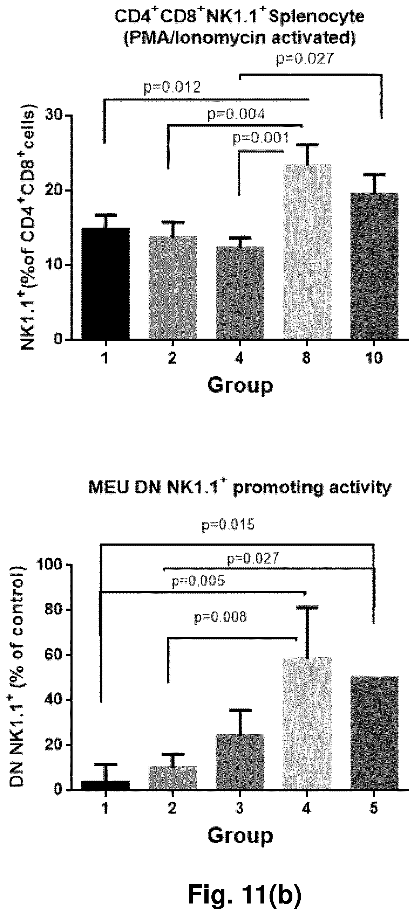

In the group 8, not only anti-MEU specific total IgG and IgG1 are increased but also, a very specific group of immune CD4⁺CD8⁺NK1.1⁺ and CD4⁻CD8⁻NK1.1⁺ cells are also increased. NK cells exhibit pathogen-specific innate memory and adaptive functions. NK cells have recently emerged as both key regulators of vaccine-elicited T and B cell responses and as memory cells that contribute to pathogen control (DOI: https://doi.org/10.1016/j.tips.2021.06.004). This phenomenon is observed in the splenocytes upon stimulation with the MEU antigen. This significant increased level is observed in the cells after stimulation with the MEU antigen and/or PMA/lonomycin as positive control. This data with the focus on the CD4⁺CD8⁺NK1.1⁺ and CD4⁻ CD8-NK1.1⁺ cells in infected mice immunised with MEU+Flagellin is depicted in Fig. 11(a)-(b).

### Example 8: Humoral immune response:

To evaluate the humoral immune response, the serum of the animals was collected, and antibodies (IgG) were analysed in an ELISA which is developed exclusively for this study.

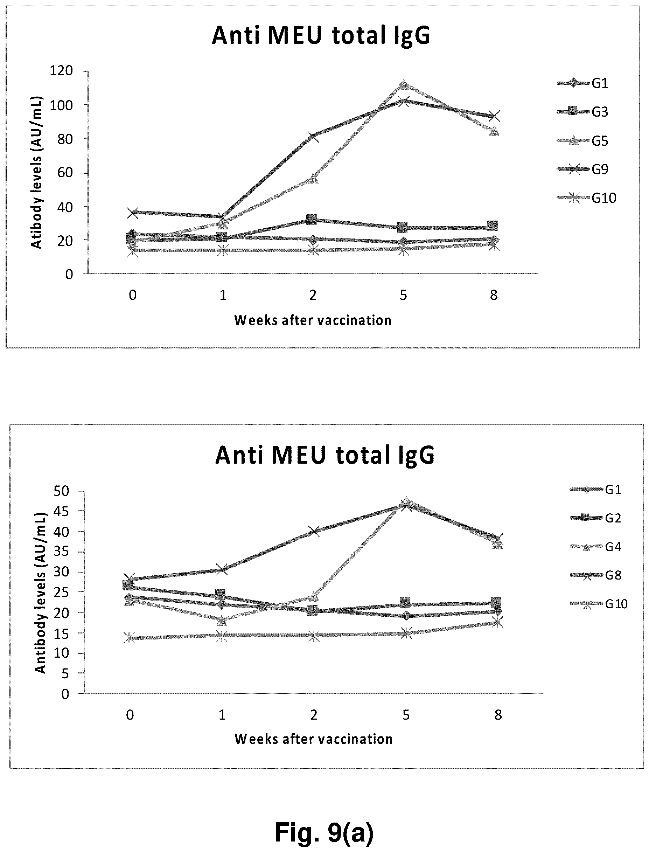

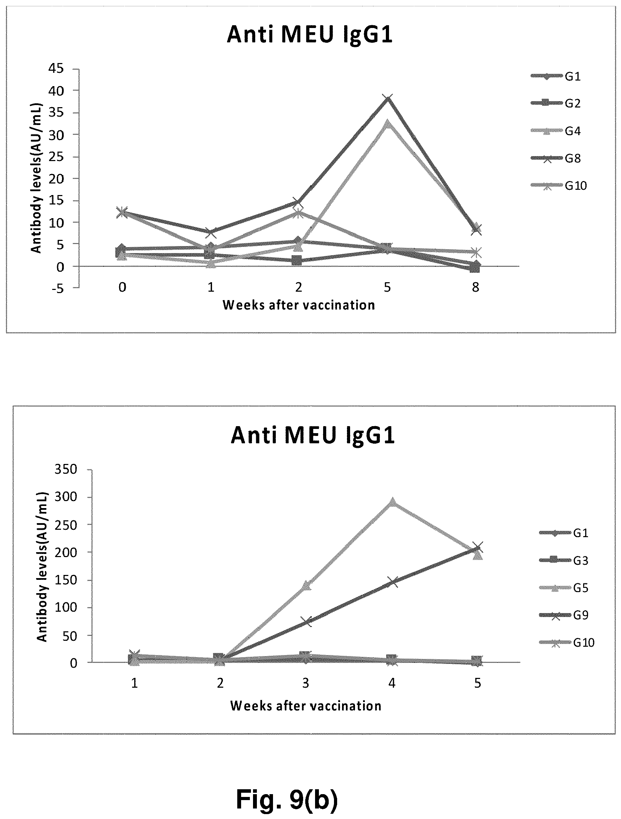

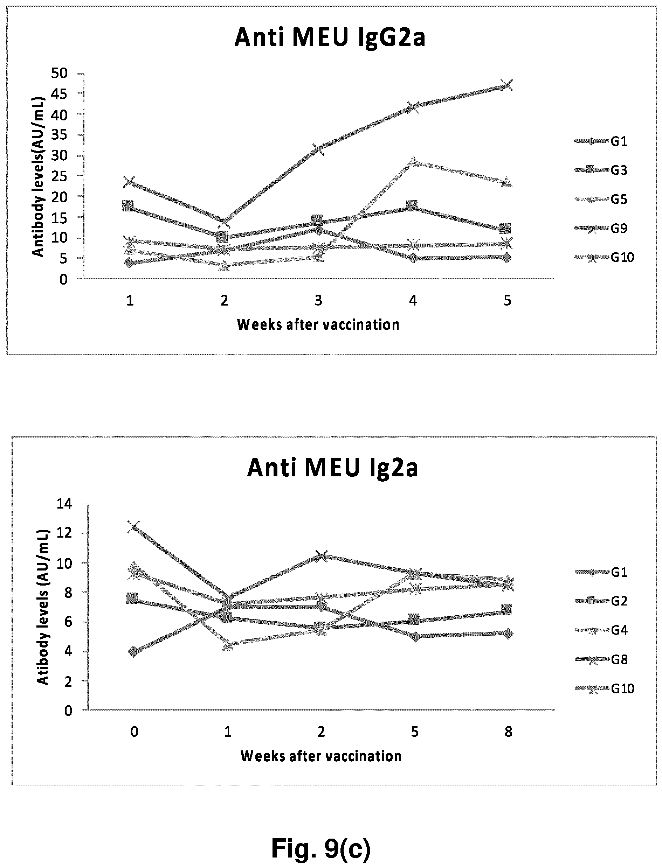

As mentioned above, the identified lower limit of detection (LLoD) of this ELISA is 1.03 AU/ml. Fig. 9(a)-(c) show the level of MEU specific antibodies in the serum of the immunised mice.

### Example 9: Avidity of the Antibodies:

In order to evaluate the binding affinity level of the antibodies to the antigen, the avidity measurement was conducted.

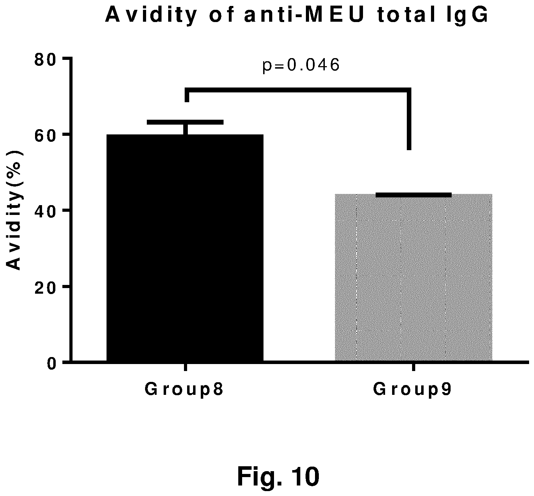

Fig. 10 shows that group 8 and 9 significantly and to some extend group 5 show reduction of bacterial load. The analysis of the cellular and humoral immune response shows differences in the immune response mainly due to the adjuvant applied.

In the animals where CTB is applied as adjuvant, the main immune response is humoral. It is observed that 3 weeks after the beginning of immunisation anti-MEU specific total IgG, IgG1 and IgG2a are increased. This response slightly drops after the 4^{th} week in the group 5 which it keeps increasing in the group 9. No significant cell response is observed in the groups 5 and 9. Moreover, the analysis of the avidity of antibodies revealed that the antibodies of group 8 have significantly higher avidity to MEU compared the antibodies of group 9. This could be a part of the explanation of the better efficacy observed on the group 8.

### Discussion

The presently disclosed is based on a new constructed synthetic antigen. It is of importance that apart from evaluation of the immunogenicity of the protein, its processing in the antigen presenting cells and reactivity of its epitopes with antibodies of naturally infected individuals is analysed.

Altogether, the MEU antigen is a potential antigen which induces cellular and humoral immune response in mice. None of the immunised animals showed visible adverse effect upon immunisation which is the preliminary indication of non-toxicity of the new vaccine. Moreover, the antibodies raised in the immunised mice can bind to the natural crude lysate of *H. pylori.* This confirms the correct antigen processing of the new antigen. In addition, the human sera from infected individuals show a significant reactivity with the new antigen. This finding demonstrates the rightness of the design of the new antigen:
1. The MEU (Multi Epitope Unit) antigen shows high expected antigenicity;
2. Cellular and antibody response due to the immunisation are achieved;
3. Preliminary indication of no toxicity (Healthy animals, no death upon immunisation);
4. Reacting of the MEU antigen with human antibodies;
5. Binding of the mouse anti-MEU antibodies to the *H. pylori's* natural crude antigens;
6. Strong antibody response in infected-Immunised animals;
7. Both T- helper-1 (Th1) and T-helper-2 (Th2) responses are observed depending to adjuvant;
8. Significant reduction of bacterial load in infected animals 4 weeks after immunisation in at least 2 groups;

## Claims

1. A polypeptide, comprising a sequence of an epitope from UreaseB, wherein said sequence of an epitope from UreaseB comprises a sequence exhibiting at least 70% identity to SEQ ID NO: 1, and/or a sequence exhibiting at least 70% identity to SEQ ID NO: 2, and/or a sequence exhibiting at least 70% identity to SEQ ID NO: 3, and/or a sequence exhibiting at least 70% identity to SEQ ID NO: 4.

2. The polypeptide according to claim 1, wherein said polypeptide further comprises a sequence of an epitope from FliD, and wherein said sequence of an epitope from FliD comprises a sequence exhibiting at least 70% identity to SEQ ID NO: 5, and/or a sequence exhibiting at least 70% identity to SEQ ID NO: 6, and/or a sequence exhibiting at least 70% identity to SEQ ID NO: 7, and/or a sequence exhibiting at least 70% identity to SEQ ID NO: 8, and/or a sequence exhibiting at least 70% identity to SEQ ID NO: 9, and/or a sequence exhibiting at least 70% identity to SEQ ID NO: 10, and/or a sequence exhibiting at least 70% identity to SEQ ID NO: 11, and/or a sequence exhibiting at least 70% identity to SEQ ID NO: 12, and/or a sequence exhibiting at least 70% identity to SEQ ID NO: 13, and/or a sequence exhibiting at least 70% identity to SEQ ID NO: 14, and/or a sequence exhibiting at least 70% identity to SEQ ID NO: 15.

3. The polypeptide according to claim 1 or claim 2, wherein said polypeptide further comprises a sequences of an epitope from HpaA, and wherein said sequence of an epitope from HpaA comprises a sequence exhibiting at least 70% identity to SEQ ID NO: 16, and/or a sequence exhibiting at least 70% identity to SEQ ID NO: 17, and/or a sequence exhibiting at least 70% identity to SEQ ID NO: 18, and/or a sequence exhibiting at least 70% identity to SEQ ID NO: 19, and/or a sequence exhibiting at least 70% identity to SEQ ID NO: 20, and/or a sequence exhibiting at least 70% identity to SEQ ID NO: 21, and/or a sequence exhibiting at least 70% identity to SEQ ID NO: 22.

4. The polypeptide according to any one of claims 1 to 3, wherein said polypeptide further comprises a sequences of an epitope from HP0231, and wherein said sequence of an epitope from HP0231 comprises a sequence exhibiting at least 70% identity to SEQ ID NO: 23, and/or a sequence exhibiting at least 70% identity to SEQ ID NO: 24, and/or a sequence exhibiting at least 70% identity to SEQ ID NO: 25, and/or a sequence exhibiting at least 70% identity to SEQ ID NO: 26, and/or a sequence exhibiting at least 70% identity to SEQ ID NO: 27.

5. The polypeptide according to any one of claims 1 to 4, wherein said polypeptide further comprises a sequence of an epitope from NapA, and wherein said sequence of an epitope from NapA comprises a sequence exhibiting at least 70% identity to SEQ ID NO: 28, and/or a sequence exhibiting at least 70% identity to SEQ ID NO: 29.

6. The polypeptide according to any one of claims 1 to 5, wherein said polypeptide further comprises a sequence of an epitope from BabA, and wherein said sequence of epitope from BabA comprises a sequence exhibiting at least 70% identity to SEQ ID NO: 30.

7. The polypeptide according to any one of claims 1 to 6, wherein said polypeptide further comprises a sequence exhibiting at least 70% identity to SEQ ID NO: 31 and/or a sequence exhibiting at least 70% identity to SEQ ID NO: 32.

8. The polypeptide according to any one of claims 1 to 7, wherein said polypeptide further comprises a sequence exhibiting at least 70% identity to SEQ ID NO: 33, and/or a sequence exhibiting at least 70% identity to SEQ ID NO: 34, and/or a sequence exhibiting at least 70% identity to SEQ ID NO: 36.

9. The polypeptide according to any one of claims 1 to 8, wherein said polypeptide comprises two or more of sequences selected from SEQ ID NO: 1-SEQ ID NO: 30, and wherein said two or more of sequences are linked to each other through a linker selected from a group consisting of KK, GGS, KFERQ and CTGKSC.

10. A polypeptide for use in preventing or treating an infection by *H. pylori* and/or a disease caused by *H. pylori* infection, wherein said polypeptide is a polypeptide according to any one of claims 1 to 9.

11. A composition comprising the polypeptide according to any one of claims 1 to 9, further comprises a separate polypeptide which comprises a sequence displaying at least 70% identity to SEQ ID NO: 31, SEQ ID NO: 32, or SEQ ID NO: 33.

12. The composition according to claim 11, further comprising a separate polypeptide which comprises a sequence displaying at least 70% identity to SEQ ID NO: 34, and/or a separate polypeptide which comprises a sequence displaying at least 70% identity to SEQ ID NO: 36.

13. A composition comprising the polypeptide according to any one of claims 1 to 9, further comprises a separate polypeptide which comprises a sequence displaying at least 70% identity to SEQ ID NO: 34, and/or a separate polypeptide which comprises a sequence displaying at least 70% identity to SEQ ID NO: 36.

14. A composition for use in in preventing or treating an infection by *H. pylori* and/or a disease caused by *H. pylori* infection, wherein said composition is a composition according to any one of claims 11 to 13.

15. The composition for use according to claim 14, wherein the composition is administered through oral administration, sublingual administration, nasal spray or intramuscular injection.
